(19) Europäisches Patentamt / European Patent Office / Office européen des brevets

(11) **EP 3 612 270 B1**

(12) **EUROPEAN PATENT SPECIFICATION**

(45) Date of publication and mention
of the grant of the patent:
**10.11.2021  Bulletin 2021/45**

(21) Application number: **18863801.9**

(22) Date of filing: **17.04.2018**

(51) Int Cl.:
*A61N 2/02* *(2006.01)*

(86) International application number:
**PCT/RO2018/000007**

(87) International publication number:
**WO 2019/117740 (20.06.2019 Gazette 2019/25)**

(54) **ELECTROMAGNETIC MODULAR SYSTEM FOR CELLULAR REGENERATION**

ELEKTROMAGNETISCHES MODULARES SYSTEM FÜR ZELLREGENERATION

SYSTÈME MODULAIRE ÉLECTROMAGNÉTIQUE POUR LA RÉGÉNÉRATION CELLULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **18.04.2017  RO 201700234**

(43) Date of publication of application:
**26.02.2020  Bulletin 2020/09**

(73) Proprietor: **Vladila, Bogdan Constantin
031165 Bucharest (RO)**

(72) Inventor: **Vladila, Bogdan Constantin
031165 Bucharest (RO)**

(74) Representative: **Tuluca, F. Doina
Cabinet Doina Tuluca
Bd. Lacul Tei 56
Bl.19, sc.B, ap 52, sector 2
020392 Bucuresti (RO)**

(56) References cited:
**WO-A1-2017/055471      RO-A- 131 383
US-A1- 2003 095 022**

## Description

The invention relates to a modular electromagnetic system for regeneration at a cellular level, which can be used especially in the medical and cosmetic fields.

Numerous experimental research has shown the influence of the electromagnetic field (EMF) on important cellular processes such as adhesion, proliferation, differentiation, directional migration, and division. There are a considerable number of publications that show the beneficial physiological effect in EMF in vivo and in vitro. Cancer, cardiac muscle regeneration, diabetes, arthritis and neurological disorders are just some of the pathologies that have been shown to be receptive to EMF therapy. Since 2004, numerous in vitro and in vivo studies and pilot clinical studies have highlighted the therapeutic potential of the electric field in various types of cancer. (Rehman et al., 2015)

The incidence of primary cerebral tumors has increased and is associated with a significant mortality and morbidity rate. Glioblastoma is the most common but most lethal form of primary tumor of the nervous system, 45.6% of all malignant tumors of the nervous system, with an average survival rate of 5 years despite of multimodal therapy. Specific treatment is adapted to each patient according to age, prognosis, location, type, degree and stage of the tumor. Aggressive surgery with total resection of glioblastoma improves the survival rate, but the complete microscopic removal of the tumor is difficult to achieve because of its infiltrative and diffuse character. Therefore, most glioblastomas are recurrent. Traditional adjuvant therapy after resection involves radiotherapy that increases the survival rate by 3-6 months. The primary initiator cytostatic is temozolamide, on the second line of cytostatic treatment being irinotecan and bevacizumab, as well as the older generation drugs - procarbazine, lomustine and vincristine. These chemotherapeutic agents are accompanied by side effects. Patients treated with temozolomide face hair loss, nausea, vomiting, headache, fatigue, anorexia, lymphocytopenia. Bevacizumab is associated with bleeding events, hypertension, perforation of the colon, thromboembolism and renal dysfunction.

The area of the bioelectromagnetic field (with different wavelengths and frequencies) offers a substantial innovative potential by exploring the effects on biological systems, including cells and tissues. Potential of the electric field was used for cancer therapy and in April 2011 the FDA approved the system NovoTTF-100A (Novocure, Inc.) as the standard therapy in recurrent glioblastoma refractory to surgical treatment and radiotherapy. Since 2009, this portable battery device by which an alternate electric field is transmitted to the tumor via electrodes applied to the scalp, is also marketed in Europe.

Also, another therapeutic option for cancer patients is pulsed electromagnetic field treatment. Literature shows that these fields do not cause predictive effects on DNA but can epigenetically act on gene expression. A study shows that the application of a pulsed electromagnetic field (2mT, 75Hz) on the T98G human glioblastoma line in combination with temozolomide induces a pro-apoptotic epigenetic effect suggesting a synergistic effect with it (Pari et al., 2016).

Another study confirms that proliferation and apoptosis of multiform glioblastoma cells (a very refractory form of treatment of any kind) are influenced by extremely low electromagnetic field exposure (Akbarnejad et al., 2016).

Electronic Doctor is a 100% Romanian, innovative, generating low frequency electromagnetic field with applications in dermatology / aesthetics and dentistry, and is the subject of patent applications RO131088 A1 published on 30.05.2016, RO130214A1 published on 29.05.2015 , RO130324 A1 published on 30.06.2015, and RO131383 (A0), published on 2016-09-30, which allows a non-invasive approach to the treatment of wrinkles, stretch marks, hair loss and periodontal diseases. Correcting data from the literature, the modulation of the operating parameters (frequency, wavelength, exposure time) can give the device new valences that can be used in the brain and even in oncology therapy. In fact, such applications have also been mentioned in previous patent applications, especially in RO131383 (A0), without emphasizing them. Also, known devices in the field generate electromagnetic pulses of very low frequency, with intensities and amplitudes sometimes significantly lower than those attributable to the terrestrial magnetism.

There are also electromagnetic field application devices that include a current component and display oscillations for the same reason, therefore the cellular effects of these devices are not the ones expected over time, and at the same time are relatively difficult for patients, particularly those with a high sensitivity that allow application only in small areas, which increases the duration of treatment. For example, International Patent Application WO 2009/04215 A1 discloses an electronic device for speeding tissue healing in the knee area by means of the low frequency electromagnetic field. The device is part of a system designed to heal the tissues and bones of various affections and is equipped with an electric circuit for controlling the magnetic field pulse.

The patent RO121463 (B1) - 2007-06-29 discloses a bioenergy rebalancing device for the human body, which contains an inductor generating electromagnetic pulses, that has a transistor-based oscillator, in which it feeds an induction coil, causing its opening and closing, the transistor being commanded in the base, also by a second oscillator, with adjustable frequency and having the value of the frequency higher than the first oscillator, so that, following the command of the two oscillators, the coil generates trains pulses, having a frequency determined by the second oscillator, and the pulse train duration determined by the first oscillator, the negative pulses being eliminated by a diode mounted in parallel on the induction coil.

It is also known the invention entitled "Magnetic stimulation device", author MAYR Winfried No. WO2017055471 (A1), published on 06.04.2017 - The invention relates to a device (1) for magnetic stimulation of regions (R) of the body, comprising at least two magnet coils configured as air coils or at least one magnet coil (2, 2') arranged on a magnet yoke, said coils being connected to a stimulator (3) which has a power unit (4) for generating electrical pulses (I) to be applied to the at least one magnet coil (2, 2'), wherein the at least two magnet coils configured as air coils or the poles of the magnet yoke can be arranged on both sides of the region of the body to be stimulated. In order to be able to align the magnetic stimulation field optimally with the respective motor stimulation points of the region (R) of the body to be stimulated, the at least two magnet coils (2, 2') configured as air coils or the poles (7, 8) of the magnet yoke (6) are adjustable by means of an adjusting device (5) relative to the region (R) of the body to be stimulated, the configuration being such that the region (R) of the body to be stimulated can be arranged within the induced magnetic field (H), wherein the adjusting device (5) includes a swivel joint (9) and/or a slide joint (10). It is also known the invention RO 131 383 A0, published on 30.09.2016. The invention relates to an Electromagnetic equipment for increasing the elasticity of tissues used in the cosmetic field, comprises a support stand (A), an applicator-coil(B) for applying the electromagnetic field, a filter (C) for filtering the harmonics of the applied current and an apparatus (D) for generating an electromagnetic field, the assembly consisting of the device (D), the filter (C) and the coil applicator (B) being constructed so as to generate inside the coil applicator (B), in an area X, a uniform electromagnetic field, induction range between 0.7-0.8 ml, with a variation between5 -10% in the area subjected to the electromagnetic field, at a distance of 1 cm around the same point, on a volume as large as possible in the areas treated, produced by a current of 400 mA, of a single frequency between 7 and 8 Hz, preferably 7,692 Hz, the field lines being perpendicular to the surface of the skin, and in which the coil applicator (B) through which the uniform electromagnetic field is applying a about, perpendicular to the area located next to them of the cellular tissue, consists of two magnetic cores, one inner (5) and one outer (6), in the form of a circle and a housing (7), around each core ( 5,6) being wound a small coil (9) and a large coil (10), respectively, coupled in series with each other, so as to allow an induction of 0.6-0.8 mT in the treatment area of the tissue, and a uniform magnetic field perpendicular to the surface to be treated, for example of the skin, the output of the large coil (10) being connected to the input of the small coil (9) and the inlet of the large coil (10) and the output of the small coil (9) being electrically connected to the apparatus (D) electronically generating electromagnetic field by means of the filter (C) for filtering the harmonics of the current delivered by the device (D).

The devices described here have the following disadvantages: they generate a single frequency domain in a therapy session, have only one channel to generate electromagnetic pulses, and require discontinuation of the patient's current activity during treatment.

The technical problem solved by the electromagnetic system according to the invention consists in the realization of a mobile modular system capable of generating the frequency of therapy on several electromagnetic pulse generation channels to produce an uniform electromagnetic field perpendicular to the treatment area.

According to the invention, the modular electromagnetic cellular regeneration system comprises a patient positioning means, an upper coil-applicator and a lower coil-applicator for applying the electromagnetic field to the desired area, a support and displacement platform for the upper coil-applicator and the lower coil-applicator and an electromagnetic field generator device with 7 outputs which is placed in a cassette and generates an alternating signal with adjustable current counting by 50mA from 0 to 600 mA, and with frequency ranging from 7 to 8 Hz, preferably 7.69 Hz, generating a sinusidal current signal which applied to the coils, generates in the space near them an uniform electromagnetic field with the induction in the range of 0.7-0.8 mT with a variation between 5 - 10% in the area with the electromagnetic field, on a distance of 1 cm around the same point, on a larger volume in aplication areas, the field lines being perpendicular to the surface of the target tissue, and wherein:

- the patient positioning means (is a bed on which the patient can sit comfortably and at the same time performs other activities - working or relaxing - during the treatment;

  - the upper coil-applicator and the lower coil-applicator generate an uniform electromagnetic field perpendicular to the localized area of their cellular tissue having the same construction and each comprising a magnetic core around which some coils are wound which are mounted inside a rectangular or circular housing,

  - the support and displacement platform of the upper coil-applicator and the lower coil-applicator consists of a guiding tower having at the top an upper platform that supports and displaces the upper coil-applicator, and at the bottom a lower platform that supports and displaces the lower coil-applicator; and

  - the device generates low frequency current signals, and is a low frequency sinusoidal current generator in the range of 2 Hz to 25 Hz with 7 outputs with a maximum output level of 600mA RMS in outputs from 1 to 7, maximum output voltage of 24 V RMS, and is built around a microprocessor and a DDS (Digital Current Synthesizer) adapted to directly generate precise sinusoidal signals with a variation from 2 to 25 Hz with a high precision and stability driven by the processor entering in some amplifier units with an adjustable current which varies from 1 to 400 mA, where both the frequency and the current are continuously controlled by the microprocessor, the outputs of the amplifying units being applied to the relevant terminals of the device which is operably connected to the coil

The electromagnetic cellular regeneration system, according to the invention has the following advantages:

- accelerates tissue repair;
- the device is well supported during the patient's treatment without any effort on their part and without interrupting their current work, for example from the office or during sleep;
- relatively simple construction;
- safety in operation.

The invention is defined in the following claims. Other embodiments, examples and methods do not constitute a part of the invention.

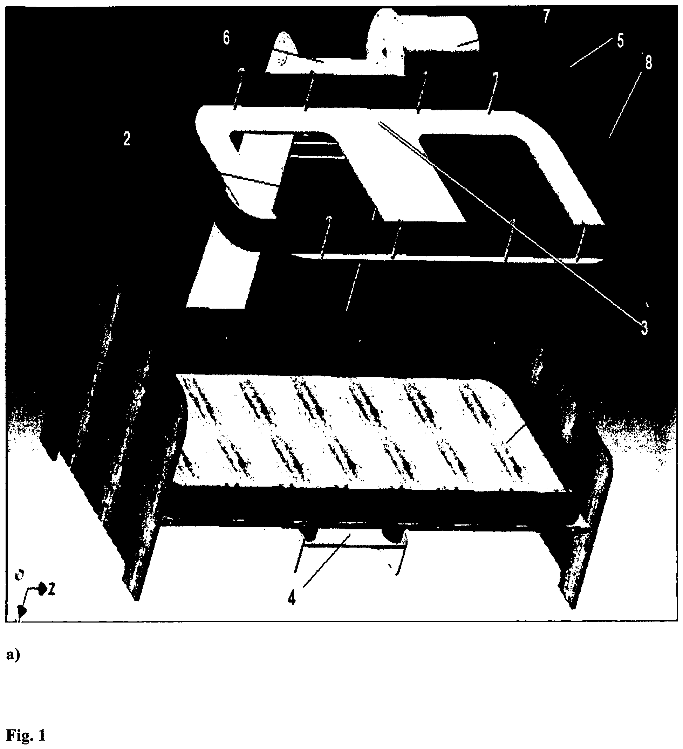

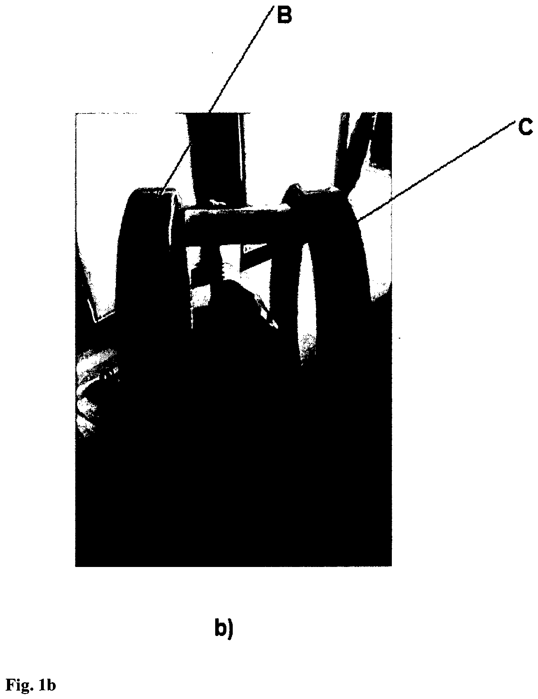

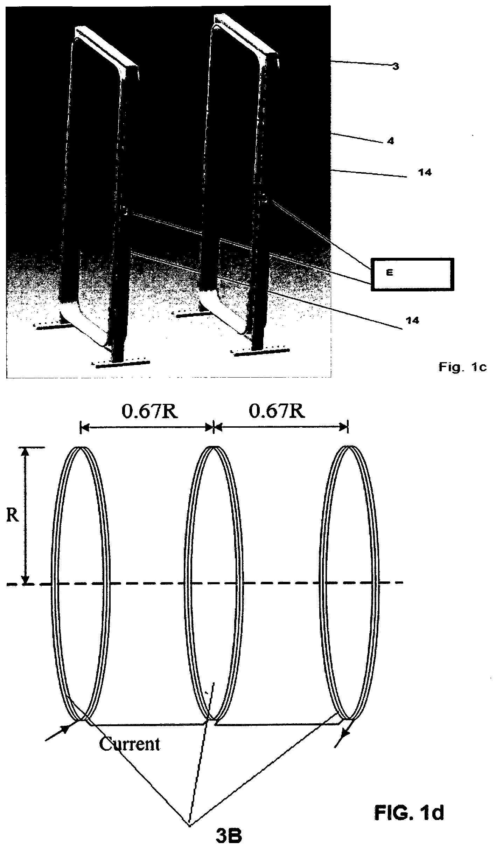

It can give the following examples of embodiments of the electromagnetic cellular regeneration system according to the invention in connection with Fig. 1 ..., which:

- fig. 1 is a general view of the system according to the invention:

    a) the first embodiment, for bed;
    b) the second embodiment, with the set of coil-applicators, a left platform and a right platform for supporting coil-applicators and electromagnetic field generating device directly connected to coil-applicators;
    c) the third embodiment, with only the set of coil-applicators and the electromagnetic field generating device E;
    d) the 4 -th embodiment with equidistant three coils assembly;

- fig. 2 shows a view of the system according to the invention:

    a) lateral
    b) from above
    c) below

- fig. 3 is a view of the system according to the invention in the operating position:

    a) lateral
    b) from above
    c) below

- fig. 4 is a view of a guiding tower of the system according to the invention;

    a) from the front
    b) lateral

- fig 5 is a view of a guiding rail with balls of the system according to the invention;

    a) from above

    b) from the front

    c) lateral

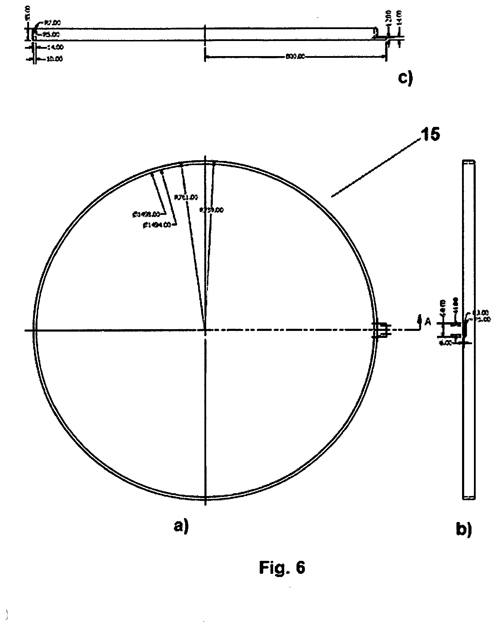

- fig. 6 is a view of a coil-applicator frame of the system according to the invention:

    a) from above

    b) from the front

    c) lateral

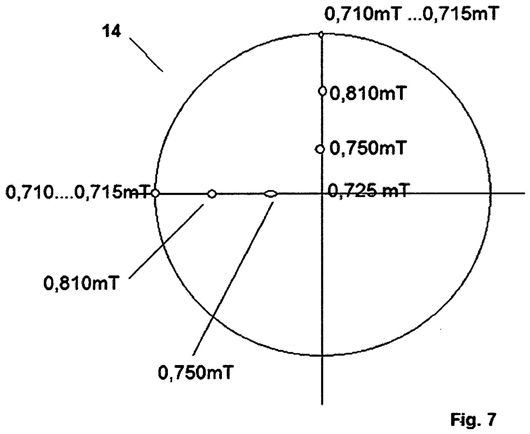

- fig. 7 is the electromagnetic field distribution at the lower part of the coil-applicators, according to an embodiment of the electromagnetic system;

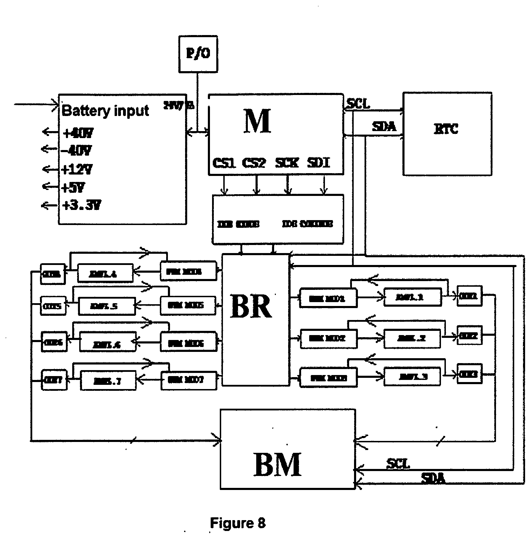

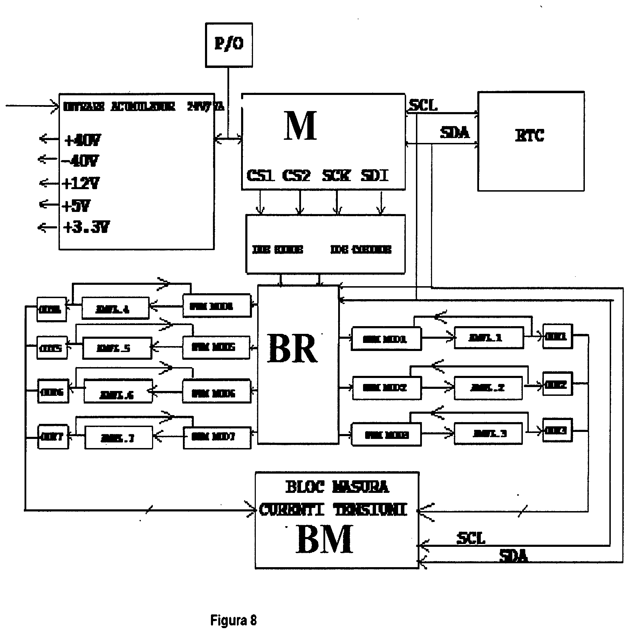

- fig. 8 is a block diagram of an electromagnetic cellular regeneration device with 7 outputs according to an embodiment of the modular electromagnetic system;

- fig. 9 is an electronic schematic of the Direct Digital Synthesizer DDS of the electromagnetic cellular regeneration device with 7 outputs, according to an embodiment of the modular electromagnetic system;

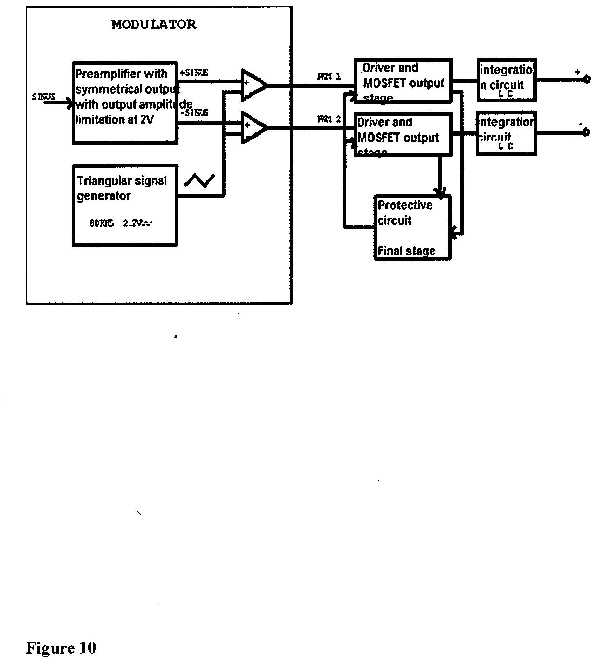

- fig. 10 is an electronic schematic of an amplification unit for the electromagnetic cellular regeneration device with 7 outputs, according to an embodiment of the modular electromagnetic system;

- fig. 1 1 shows the modulated signal obtained after passing through the comparators of an amplification unit for the electromagnetic cellular regeneration device with 7 outputs according to an embodiment of the modular electromagnetic system;

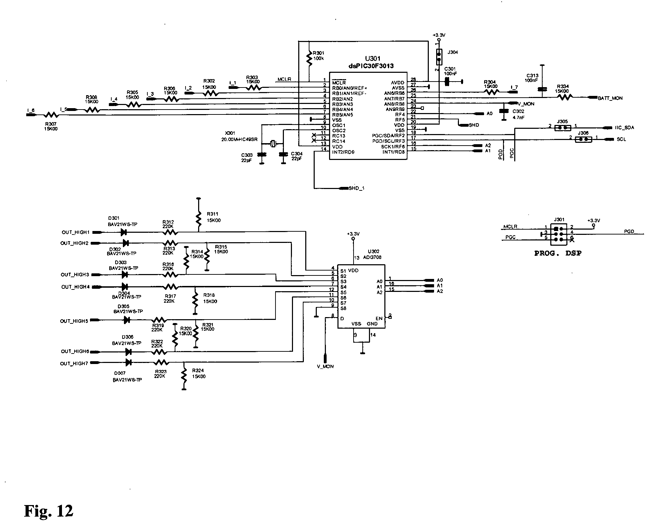

- fig. 12 is an electronic schematic of the measure block of the electromagnetic cellular regeneration device according to an embodiment of the modular electromagnetic system;

The electromagnetic cellular regeneration system according to a first embodiment of the invention, Figure 1a, comprises a patient positioning means **A**, an upper coil-applicator **B** and a lower coil-applicator **C** for applying the electromagnetic field to the desired area, a support and displacement platform **B** for the upper coil-applicator **B** and the lower coil-applicator **C** and a device **E** for generating an electromagnetic field with 7 outputs which is placed in a cassette **7.** Applicators **B** and **C** form a Helmholtz coil assembly. If the device **E** generates a current set at 100 mA, with a frequency of 7 to 8 Hz, preferably 7.69 Hz, this is transmitted to an amplifier, then from its output is applied to coils **B** and **C** generating, in the space between them, an uniform electromagnetic field with the induction in the range of 0.7-0.8 mT with a variation between 5 - 10% in the area under electromagnetic field , on a distance of 1cm around a single point, on a larger volume in the application areas, producing a current, the field lines being perpendicular to the surface of the target tissue. At currents above 600 mA to 6 A, outputs from the amplifier units (**AMPL1-7**) are applied to the relevant terminals of the device (**E**) via another amplifier, not shown.

Patient positioning means **A** may be a bed **1,** e.g. a hospital bed, as shown in Figure Ia, or an office chair, hairdresser, or something like that, or any means of positioning the patient, so that the patient can sit comfortably and at the same time performs other activities - work or relax - during the treatment.

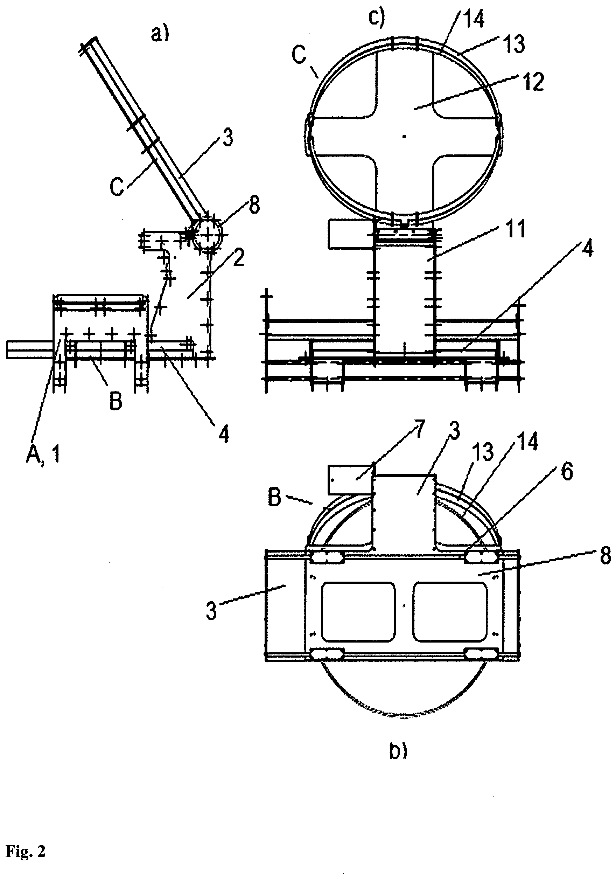

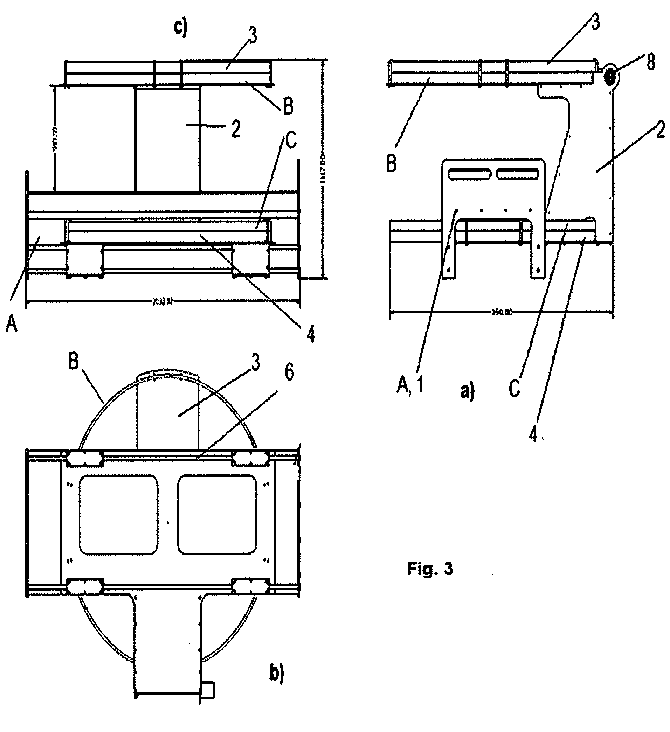

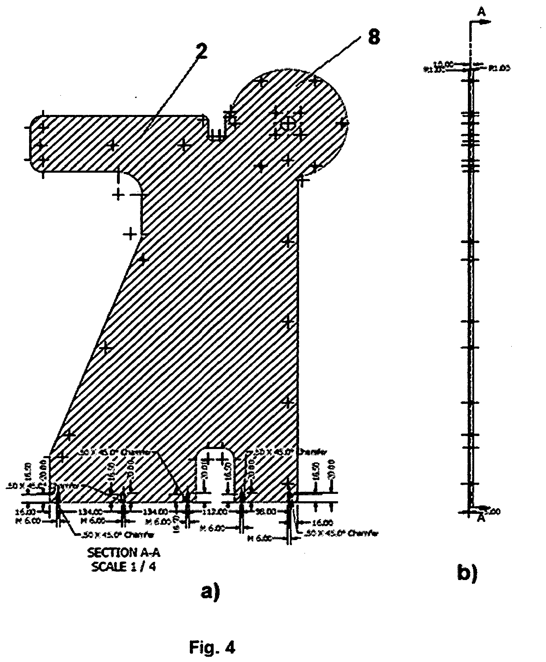

According to a first embodiment, when the means **A** is a bed, the support and displacement platform **D** of the upper coil-applicator **B** and the lower coil-applicator **C** from Figures 2 and 3 is formed by a guide tower **2,** figure 4, which at the top has an upper platform **3** for supporting and displacing the upper coil-applicator **B,** and at the bottom, a platform **4** for supporting and displacing the lower coil-applicator **C.**

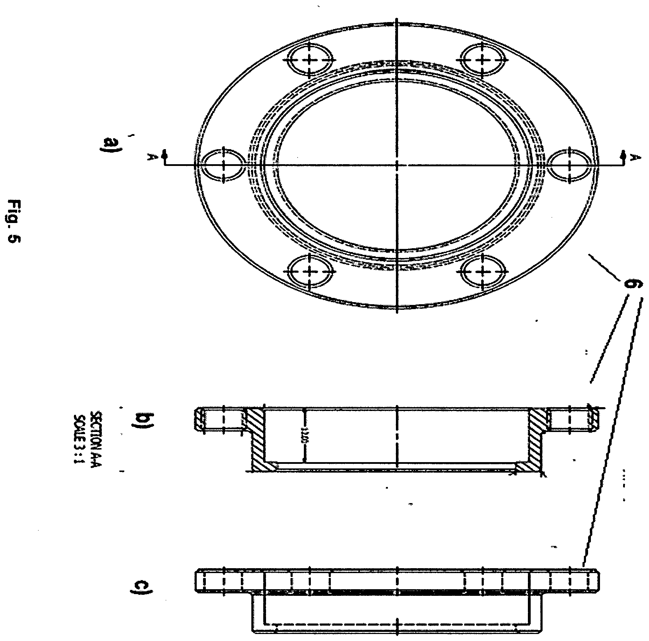

In order to allow the upper coil-applicator **B** to be lifted or lowered by the mobile platform **3** if the system according to the invention is operated or not and to be moved over the entire width of the tower **2,** this is placed at the top of the tower **2** by a guiding assembly **5** shown in Figure 5. The guiding assembly **5** lifting and / or the lowering the upper platform **3** is provided with a displacement rail **6** on balls. The movement of the rail **6** is accomplished with the help of an electric step by step motor (not shown) which is inside a cassette **7.** According to another example, the upper platform **3** can also be moved manually. The upper coil-applicator **B** is fastened with the help of an upper frame **8** of the upper platform **3.** The fastening is made with the help of known fastening means, for example, bolts, nuts or something simillar. Also, fastening the upper platform **3** to the tower **2** is accomplished by some ball bearings **9** which allow the upper frame **8** to be lowered / lifted.

In the lower part, the lower platform **4** for the supporting and the guide of the coil-applicator **C** across the width of the tower 2, is placed at the bottom of the tower **2** with the help of a guiding assembly **10** shown in figures 2c and 3c. The guide assembly **10** of the lower platform **4** is provided with the guide rail **11** which is connected to the guide rail **6** of the upper platform **3,** so with the displacement of the upper part, it also moves the lower coil-applicator **C.** The lower platform **4** is provided with a fastened frame **12** of the lower coil-applicator **C** . The fastened of the upper coil-applicator **B** with the mobile frame **12** is accomplished known fastened means, e.g., bolts, nut bolts or something simillar. Also, the upper platform **4** is fastened to the tower **2** with the help of the guide rail **11** provided with some channels (not shown).

The tower **2,** the upper platform **3** and the lower platform **4** are made, without limiting to those, of 10 mm thick sticlostratitex material so it will not deform the magnetic field around the upper and lower applicators **B,** respectively **C.** The fastened screws can be made of stainless steel because their influence on the magnetic field is minimal.

The upper coil-applicator **B** and the lower coil-applicator **C** generate an uniform electromagnetic field perpendicular to the localized area near them of the cellular tissue. They have the same construction and can be made in two forms: round and rectangular.

Coil-applicators **B** and respectively **C** each have in essence a rectangular or circular magnetic core **13** around in which are convoluted **14** coils, which are placed, inside a rectangular or circular frame **15.**

According to a embodiment, the magnetic core **13** on which the coil **14** is wrapped over is made of 5 to 10 mm aluminum, 100-120 cm long, preferably 119 cm, 10 cm width and 29 to 33 cm in diameter, preferably 30 cm.

According to of one embodiment, the coils **14** are made of enamelled copper wire having a coil height of 20 -130 mm, preferably 100 mm, a length of 500-2000 mm, preferably 1982mm, a width of: 100-700 mm , preferably 682 mm, a 1.2 mm thickness of the convoluted wire, a number of whirls 100-500, preferably 460, having the diameter of the spool on which it convolutes at 100-2000mm, preferably 1538mm, a distance between coils of 100 to 1000 mm, preferably 750 mm. The coils **14** are connected in pararel (start with start, end with end). The frame **15** is preferably made of glass-tectololite material.

What is very important for the present invention is that all components of the system are modular, which allows them to be mounted and dismantled at the patient's home, at the workplace, or at other locations required by the patient.

According to another embodiment shown in figure 1c, the cellular regeneration system according to the invention comprises the set of coil-applicators **B** and respectively **C,** a left platform **3** and a right platform **4** for supporting coil-applicators **B** and respectively **C,** and electromagnetic field generating device **E** directly connected by electrical connectors to coil-applicators **B** and respectively **C.** The device **E** generates a sinusoidal current signal and is set on a 100mA current with a frequency of 7 to 8 Hz, preferably 7.69 Hz, which is transmitted to an amplifier and then from the output of the amplifier is applied to the coils **B** and **C** generating in the space between them, an uniform electromagnetic field with the induction in the range of 0.7-0.8 mT, with a variation between 5 - 10% in the area under electromagnetic field, over a distance of 1 cm in which the same point, on a larger volume in the application areas, produced a current, the field lines being perpendicular to the surface of the target tissue. The coils **B** and respectively **C** can be placed to the left and right of the table, the bed, the seat, etc and still obeys the helmholtz rule. For example, if the table has a length of 80 cm, coils with a height of 1.60 m are used. Thus the patient is normally at the table and eats for two hours or works, and the coils do their job. This option is simple to achieve, for example, resulting in a office with coils for maintaining health. This will record the hours at which the patient is eating and the treatment can be correlated in his or her rhythm. This option can be used to left-right from hospital beds, some chairs, for example, cosmetic chairs, tables, desks, etc.

According to another embodiment shown in figure lb, the cellular regeneration system according to the invention contains only the set of coil-applicators **B** and respectively **C** and the electromagnetic field generating device **E.** This option can be used in cellular culture incubators or can be used on the knees. In this case, the size is smaller. For example, a coil **14** having a width of 50mm, a diameter of 310mm, a 1.2 mm thickness of the convoluted wire, a number of whirls of 500, a distance of 140mm between coils is used. The coils **14** are connected in series (start with end).

According to another embodiment shown in figure 1d, the cellular regeneration system according to the invention contains an equidistant three coils assembly **3B** and the electromagnetic field generating device **E.** This option determines greater magnetic field uniformity and can be used in any area, giving better results. For example, it presented below a Three Coils Helmholtz Coil Design& Calculus. If we consider a number of n identical collinear coils, situated at the same distance one of each other, as Figure 1d, and this distance being smaller then coil radius, $r$, then, the medium value for the induction on the coils axis, in a vicinity of two consecutive coils will be:

$$\vec{B} = \sum_{i=1}^{n} \vec{B}_i \qquad (1)$$

where $\overline{B}_i$ is the induction generated by a single coil. If we consider n = 3, in the equation (1), then:

$$\vec{B} = 3\vec{B}_i \qquad (3)$$

For a two coils assembly (Helmholtz configuration), situated at distance $r$ one of other, the half distance $(\dfrac{r}{2})$ axial magnetic field will be given by:

$$B = \left(\frac{4}{5}\right)^{\frac{3}{2}} \frac{\mu_0 n I}{r} \qquad (4)$$

where $\mu_0$ is the vacuum permeability, $= 4\pi \cdot 10^{-7}$ [TmA$^{-1}$]

n is coil number of turns

I = current intensity through a coil [A]

Following the results of the ANSIS simulation for three equidistant coils (Figure 1d), situated at 0.67*r* one of each other, the induction $\overline{B}$ value in a vicinity of two consecutive coils will be:

$$B = 1.0892 \cdot 10^{-6} \frac{nI}{r} \quad (3)$$

The needed module value for the induction |$\overline{B}$| is:

$$\left| \vec{B} \right| = 0{,}775 \text{mT}$$

The following input values are given:

The current value through all coils, I = 600mA (0.6A)
The coil radius, r = 30cm (0.3m)
The coil length, 1 = 5cm

From the equation (3) we have:

$$n = \frac{Br}{1.0892 \cdot 10^{-6} I} \quad (4)$$

*n* = 356.913turns, n ≈ 357turns/coil

Winding calculus

The inductance o single coil, neglecting the mutual inductance of the two coils due to the very low operating frequency is given by the equation (6)

$$L = \mu_0 n^2 \frac{A}{l} K_{LS} \quad (5)$$

Where $\mu_0$ is the vacuum magnetic permeability, *A* is the surface coil area and *I* is the coil length and $K_{LS}$ is Nagaoka coefficient (non dimensional); in order to determinate the Nagaoka coefficient for short coils we will use the Lundin's formula bellow:

$$K_{LS} = \frac{2}{\pi}\left(\frac{l}{D}\right)\left\{ \frac{\left[\ln\left(\frac{4D}{l}\right) - \frac{1}{2}\right]\left[1 + 0.383901\left(\frac{l}{D}\right)^2 + 0.017108\left(\frac{l}{D}\right)^4\right]}{\left[1 + 0.258952\left(\frac{l}{D}\right)^2\right]} + 0.093842\left(\frac{l}{D}\right)^2 + 0.0029\left(\frac{l}{D}\right)^4 - 0.000801\left(\frac{l}{D}\right)^6 \right\} \quad [6]$$

$$K_{LS} = 0.179324723$$

$$\mu_0 = 4\pi \cdot 10^{-7} \text{ [T·m·A}^{-1}]$$

$$A = \pi \cdot R^2 = 0.283\text{m}^2$$

$L$ = 0.16256H, or 162.5mH.

The inductive reactance of a coil is given by the equation:

$$X_{\text{L}} = \omega L, \qquad (7)$$

In which $\omega$ is angular velocity;

$$\omega = 2\pi v \qquad (8)$$

$v$ is current frequency [Hz]

For $v$ = 7,86Hz, $X_{\text{L}}$ = 8.03Ω

Considering the capacitive inductance sufficiently small to be neglected, the total impedance of one coil is given by the equation:

$$Z_L = \sqrt{R_C^2 + X_L^2} \qquad (9)$$

In which $R_C$ is the electrical resistance of copper winding.

$$R_C = \rho \frac{l_w}{S_w} \ [\Omega] \qquad (10)$$

Where $\rho$ is the copper resistivity ($1.72 \cdot 10^{-8}$Ωm), $l_w$ is the length of the winding wire and $S_w$ is the area surface of copper wire.

In order to calculate the copper wire diameter, we use the following relation:

$$d_w = 0.75\sqrt{I} \quad [\text{mm}] \qquad (11)$$

$d_w$ = 0.581mm

We choose a 0.6mm diameter ($d_w$) copper wire;

$$l_w = 2\pi R_m \cdot n \qquad (12)$$

In the equation (12), $R_m$ is the average radius of the coil, given by:

$$R_m = \frac{R_M + R}{2} \qquad (13)$$

To calculate $R_M$ we have to estimate the number of layer for the coil. The copper wire diameter (insulation included) is $dw_i$ = 0.615mm. In the layer thickness formula, we have to consider interlayer insulation, made from PET winding material, with a thickness $t_i$ of 0.1mm, so the total thickness for 1 layer is $t_1$ = 0.715mm.

The number of turns per layer $n_l$ is given by:

$$n_l = \frac{l}{d_i} \qquad (14)$$

$n_l$ = 81 turns/layer

The number of layers is:

$$N_l = \frac{n}{n_l} \qquad (15)$$

$N_l$ = 4.407, so $N_l$ = 5 layers

The total thickness of the coil, $T_c$ is:

$$T_c = d_{wi} \cdot N_l + (N_l - 1) \cdot t_i \qquad (16)$$

$T_c$ = 3.475mm = 0.003475m

Following this data,

$$R_M = R + T_c = 0.303475\text{m}$$

$R_m$ = 0.3017375m $l_w$ = 676.827m

$$S_w = \pi \frac{d_w^2}{4},$$

in which $d$ is copper conductor diameter $S_w$ = 2.827.$10^{-7}$m$^2$

$R_C$ = 41.173Ω

Following the equation (9) we find for $Z$:

$Z_L$ = 41.949Ω

The total impedance is given by:

$Z_T = nZ_L$, where $n$ is number of coils. In this case, $n$ = 3.

$Z_T$ = 125.847Ω

The voltage fall over one coil $U$ will be:

$$U^{\text{ef}} = Z \cdot I \qquad (17)$$

$U^{\text{ef}}$ = 75.5082V$^{\text{ef}}$ ≈ 76V$^{\text{ef}}$ The total necessary power is given by:

$$P_T = \frac{\left(U^{ef}\right)^2}{Z_T} \qquad (18)$$

$P_T$ = 45.305W ≈ 46W

Copper mass calculus

$$m_C = \rho_{\text{MCu}} \cdot l_w \cdot S_w \qquad (17)$$

$\rho_{\text{MCu}}$ = 8892kg·m$^{-3}$

$m_C$ = 1.701Kg/coil

The upper coil-applicators B and C or equidistant three coils assembly 3B are directly connected by electrical connectors to the sine wave generating device E and set on a 400 mA current.

The very low frequency electromagnetic field bioreactor treatment emitted by the device E is performed by the upper coil-applicator **B** and the lower coil-applicator **C.**

The device **E** is built around an microprocessor **M.** Considering communication requirements with a computer, you can use, for example, an ARM Cortex-M3 microprocessor that does not require BOOT LOADER for programming, download of the program does by USB, like download a memory stick directly under Windows. This microprocessor **M** must have I2C and SPI communication systems and a miniature capsule (LQFP48).

With the device **E,** the field frequency ELF is fixed, and its intensity at the target area/areas is substantially 0.75 mT, so its intensity may be somewhat higher at target tissue level will be potentially up to 3mT.

Thus, in order to obtain an extremely low frequency sinusoidal signal, according to a first embodiment of the circuits of the device **E,** it also contains a **DDS** - Current Synthesizer adapted to directly generate precise sinusoidal signals with a variation of 2 at 50 Hz. The signal generated by **DDS** has high precision and stability driven by the microprocessor **M.** The sinusoidal signals generated by **DDS** enter in some amplification units **AMPL1-7** with an adjustable current that varies from 1 to 600mA, settable from 50mA in 50mA. Both the frequency and the current are continuously controlled by the microprocessor **M.** The outputs from amplification units **AMPL1-7** are applied to relevant terminals of the device **E** that can be functionally connected to an another amplifier **AMPL** used to generate a signal greater than 600mA reaching up to 6A and at its terminals are connected the upper and lower coil applicators **B** and **C.** For the control of the device **E,** are used the following types of signals generated by the microprocessor **M:**

- communication system I2C (SCL pin 19, SDA pin 20) / P9

- communication system SPI (SCK pin 31, SDI pin 30, CSI pin 21, CS2 pin 22) / P9

- to switch on and off the device, the pin 13 / P9 digital input is used.

The synthesizer **DDS** can be an integrated circuit, for example an AD9834 circuit that generates symmetric sinusoidal signals and allows genrating of the output galvanic current to output through differential amplifier units the **AMPL1-7,** thus avoiding capacitive couplings caused by the very low-frequency. There is also the possibility of adjusting the output level through an output current regulating block **REGL,** thus setting the output current levels.

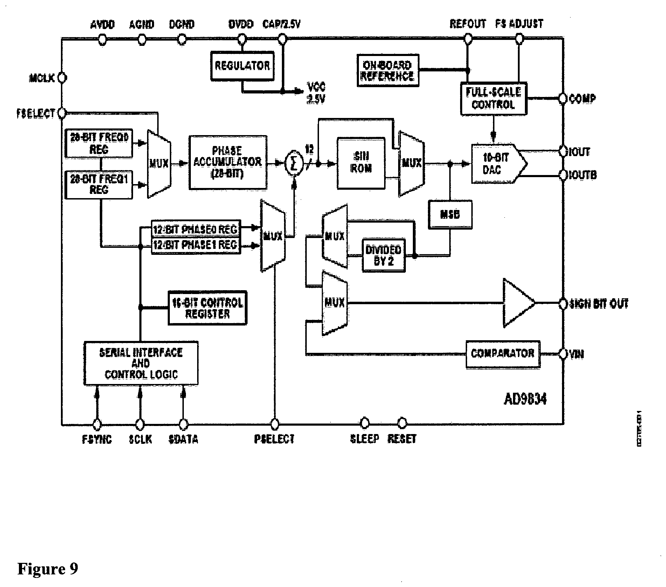

The integrated circuit of the synthesizer **DDS,** shown in Figure 9, has two frequency registers $R_{frecv}$ and two phase registers $R_{faza}$ where two frequencies and two phases can be written in an interface system **SPI.** In our case, only the frequency register $R_{frecv}$ is set. The sinus table is in SIN ROM and its run will be at the written frequency with a clock **CK.** For example, an integrated clock generator 7W-25,000 MBAT of 25 Mhz is used or the clock signal is obtained with the processor **M** by dividing the quartz crystal by 12 Mhz by $2^{10}$ and we get 11718,75 Hz which is the reference clock of the synthesizer **DDS.**

The resolution of the output current frequency is the ratio between the clock frequency and the frequency register 228 Rez = 11718,75 / 228 = 0,000043655 Hz.

Note that the output signal is not obtained by dividing the clock but by reading the SIN ROM with a sequence given by the frequency register $R_{frecv}$ and the clock **CK,** and the data is transferred by an analog converter **CDA,** at the output obtaining a sinusoidal signal from a broken line with the resolution of 12 bits truncated to 10 by the converter.

Since the working frequency is very low, it does not count on average power but on instant power. With 7 outputs, it is attempted to reduce the power peaks by making two output groups, each of which is powered by a DDS that generates synchronous signals but offset to 90 degrees.

Amplification units **AMPL 1-7** can each contain a linear amplifier, but its efficiency is up to 56% when the output voltage has a value equal to the supply voltage, but in this case, where the impedance has different values, it results that the efficiency it is between 0 and 56%, that is not allowed because it is not possible to ensure the operating autonomy and the heat evacuation.

Therefore, amplifiers **AMPL 1-7** amplifiers are used in class D (switching). The power requirement on the channel is at least 24 x 0.6 = 14.4 W RMS.

For example, according to one embodiment, for amplification units **AMPL 1-7,** an halfbridge amplifier with simmetrical feed is used. Supply voltage Va = 2 * 24 * V2 / 6, where 6 is the maximum fill factor wich we choose 90%. Va = 2 * 37.6 V DC. We will choose a ± 40 VDC cover. For amplification units **AMPL 1-7,** for example, the International Rectifier, the driver circuit dedicated with PWM IRS20957 providing with short-circuit or overcurrent protection, and the two capsules IRFI 4019 Mos FETs dedicated to digital amplifiers. A possible scheme for an amplification unit is shown in Figure 26.

To attack the digital amplifier, a PWM signal must be generated with modulators **PMW 1-7** that include the current response and PWM limitation. The high frequency triangular signal (120Khz) is used to compare the sinusoidal signal generated by the **DDSs,** so that the input signal is converted into a rectangular signal with variable filling factor signal containing the input signal information. This signal is amplified and has the amplitude of the supply voltage, the only losses being on the resistance of the MOS FETs, when they are open. As a rule, resistances are of the order m$\Omega$,

so the losses are negligible.

This signal is passed through a low pass filter **LC** and the input signal is restored.

Finally, amplifiers **AMPL 1-7** are constant current generators, the voltage for the current response being picked up on series resistors **Rs** with an applicator, not shown, and summed with an adjustable voltage given by **DDSs** that will represent the level of current. For this adjustment a control block **BJ** is used, which according to one embodiment can be a digital potentiometer type AD 5242, which are two on the chip and by the hardware configuration of address, AD0, AD1 allow communication with maximum 4 chips (only 7 potentiometers out of 8 are used).

Communication with microprocessor **M** is done on I2C (SCL, SDA).

Caused by the very low frequency, coupling levels from **DDS** to finish is galvanic and a reference voltage of 1.65 v is chosen.

Sinusoidal signals generated, triangulation signals generated and the measured current will be focused on this reference voltage.

The electromagnetic field generating device **E** is further provided with the supply sources **SA,** so according to an embodiment of the invention, the supply voltage of a cell LI Ion to ease the charging system if more batteries are connected in parallel. Since amplifiers **AMPL 1-7** have to charge maximum supply voltage (24V RMS), the instantaneous power = 75 * 5 * 2 * cos (fi) W is important because the working frequency is 7, ... HZ. Considering that the supply voltage is high and the filtering capacities in the amplifier are 4000$\mu$F and the RMS power factor can be considered the significant load reactant. Choose a 300-500W source with high-voltage start up, and the amplifier engages the load after high voltage. Also, the degree of protection is K3.

The real-time clock generator **GTC** is used to output the output signal. For example, the usual **RTCs** for example DS1337 are selected. Communication with the microprocessor **M** will be I2C (SCL, SDA).

For measuring currents and voltages, a current / voltage block **BM** is used.

For example, seven currents, seven voltages and battery voltage are measured. Since the microprocessor **M** can not satisfy this large measure range with few free analog inputs (due to the Touch Screen), a **DSP**-type microcontroller is used to process the measurement and transmission through the I2C system of the microprocessor **M.** It chooses the microcontroller DSPIC 30F3013. 7 analogue inputs are used to measure the currents by digital filtering and the voltages are measured by means of a single analogue multiplexer. The DSPIC is also programmed to generate the selection signals for multiplexer and to transmit the measured channel to the microprocessor **M.**

For the output currents adjustment, a current control circuit **BM** is used which is realized with digital potentiometers 11202, 11210, 11215 and U222 and with which the sinusoidal signal which represents the level of the output current to the modulator **PWM** is regulated.

The electromagnetic field generating device **E** functions as follows:

Microprocessor **M** is connected via the **P8** and **P9** connectors to the baseboard and the generated signals are located on the pins of the **P9** connector, Figure 22 and 23. The on-off button connects to the **J503** connector (pin **1.2**). When the button is pressed via the diode **D501,** the + 5V source is switched on with the Power signal feeding the microprocessor **M.** On start, it generates a voltage of +3.3 V at pin **3,** which by means of the **Q501** makes automatic latching of the source and the device starts.

On switch-off, by pressing the button, the + 3.3V voltage applied to pin **13** falls to 0, and the microprocessor **M** senses the off command.

The RTC clock generator is designed, for example, with U501 (DS1337) integrated circuits and communicates with the microprocessor **M** via SCL SDA.

The microprocessor **M** is connected to a **ST** temperature sensor, such as an U502 integrated circuit that communicates with the microprocessor **M** via I2C (SCL, SDA) by transmitting the temperature to the baseboard. The Microprocessor **M** decides to stop amplifiers **AMPL 1-7** if the temperature is greater than 50 degrees and warns "DEVICE OVERHEATED" on the display. If the temperature does not drop and exceeds 55 degrees, the microprocessor warns with the message "HARDWARE FAULT" and stops the power supply.

The **DDS,** represented for example by the U201, U211 (AD9834) integrated circuits generating the sinus_1 and sinus_2 signals with 90 grade and U234 and U235 operational circuits are brought to the IVpp level and focused on the reference voltage of 1.65 V.

The modulator **PWM 1-7** consists of the triangular signal generator realized with the U229 and U230 integrated circuits used in the **PWM** comparators. The signal has amplitude of 1, 5Vpp and focused on 1.65V. We detail the channel 1, the rest being the same. T

The voltage generated by the output current is picked up on the R204 resistance **Rs** and amplified and focused on the operational circuit U204 of the amplifier **unit AMPL 1.** It is further summed up with the sinusoidal signal on the U203 integrated circuit, The current difference of U203 is limited by the R207 divisor, R201 at the value of 1.48Vpp to avoid overmodulation of the PWM. By this limitation, the modulation does not exceed 90%.

With this signal and the triangular applied to the comparator U205 we obtain the PWM for the final stage. For the amplification unit, an embodiment of the unit **AMPL 1** is described, the remainder being the same.

The integrated circuit U101 (IRS20957) is for command of the IRFI4019 output transistor. It has one PWM signal input hugging internal command for LOW driver and HIGH driver. It also has a delay circuit that generates Death Time to avoid conducting the transistors at the same time. It also it has a protection circuit that detects the voltage on the internal resistance of the output transistor when it is open and exceeds the value set in pin **7** at OCSET interrupts the transistor conduction.

The BOOTSTRAP voltage for supplying the HIGH driver is obtained with diode **D103** and **C109**.

Supply voltages for the integrated circuit + 10V is obtained from the positive source with the resistance **Rs** R101 and stabilized with internal Zenner diodes of the integrated circuit, +12 V against -45 V is obtained with the resistance **R113** with respect to the mass and **D105** diodes. The PWM signal amplified at the supply voltage value ± 45VDC is filtered with the low pass filter **LC** L101 and C108 which integrates the shape of the sinus.

The integrated circuit that makes the measurement is the U301 **DSP.** Voltages collected on the measuring resistors in the amplifier unit circuit are brought to the analog-digital inputs of the **DSP.** The output voltages are rectified and selected with the U302 multiplexer and transmitted to the **DSP.** Battery voltage is divided and transmitted to this **DSP.** All values measured by the **DSP** are in the form of a vector and transmitted to the microprocessor via the I2C communication system.

The **DSP** also generates the selection sequences (A0, A1, A2) for the voltage measures.

The power sources **SA** and the power source **SP** are built with the integrated LM5030 control circuit. This circuit also contains the voltage regulator with input to pin **2** (FB) which modulates the control signal fill factor for the **Q401, Q402** switching transistors, thus maintaining the output voltage to the value programmed by the **R416, R417** and **R418** divider.

This circuit also has overcurrent protection and the signal is picked from the resistors **R409** and **R414** and applied to the pin **8** (CS). Second, to obtain the voltages are used two rectifiers and a low pass filter **LC.**

For + 12V source, it is uses the integrated circuit with 12-volt internal regulator LM2672-12 which also has the on-off command. After the microprocessor **M** has set all the functions for the operation of the apparatus **D,** it sends the source start command via the **DSP** (SHD).

With this source the cooling fans are also supplied.

For the + 5V source, this is done with the integrated circuit LM2596, which can be switching power 3A to provide power to the microprocessor **M** and 3.3 V circuits via the 3.3 volt source.

The start command is given by the start-up touch and microprocessor controlled.

For the 3.3 volt source, a linear source is chosen because the consumption is small and the switching source is not justified.

The device is powered by the external battery pack (not shown).

The microprocessor M has software which has the function of limiting the period of use of the system according to the invention to the period prescribed by the specialist but not more than two hours on day, up to 100 hours for cosmetic applications and up to 200 hours for medical applications. Also through the software implemented on the microprocessor **M,** it is ordered to close the power supply after one or two hours of use of the system according to the invention, depending on the desired application.

The software implemented on the microprocessor **M** commands:

- limiting the period of use of the system according to the invention to the period prescribed by the physician, but not more than two hours a day, for a maximum of 100 to 200 hours for medical applications;

- the interruption of 12-24 hours of power or signal generation after one or two hours of daily use of the equipment according to the invention, depending on the desired application: cosmetic or medical;

- the total power or signal generation interruption after 100 to 200 hours of use for medical applications;

- feeding the equipment or restarting the signal generation according to the invention for a new 100-hour packet after entering a user code provided by the manufacturer via a client software application linked to the microprocessor of the generator and implemented therein.

- the command start / restart the displacement of the coil-applicators **B** and respectively **C.**

The electromagnetic system for cellular regeneration at cellular level can be used, in particular, in the medical and cosmetic fields.

For example, in the medical field it can be used in, but not limited to:

- nervous regeneration, eg parkinsonism, dementia, depression, Altzheimer, multiple sclerosis, repairing the myelin sheath of the nerves;

- dry eye syndrome, dry mouth, regulation of nasal secretion;

- treatment of burns and injuries;
  treatment of hemorrhoids and varicose veins;

- treating genital diseases of the cervix, ovary and testicles;

- tissue reinforcement in the ligaments represented by the articular discs (temporomandibular joint, knee), but also in other applications involving improvement of elasticity as well as growth, regulation of tissue microcirculation, eg ENT - rhinitis, sinusitis, hearing impairment, odor, taste , vision (hypermetropia, myopia, presbytism);

- reduction of intraocular pressure that will also lead to reduction or delay of glaucoma, decrease of myopia;

- slows down cancer by strengthening healthy tissue around cancer. So advancement is slower and does not malign;

- reduces distal effects after radiotherapy and chemotherapy;

- quick recovery of athletes, recovery after intense effort and the possibility to compete after a shorter rest time;

- Increases fertility in women (either in vitro or natural fertility).

- Plus recovery after injuries, osteoporosis.

In the cosmetic field it is possible to use in, but not limited to, wrinkle reduction, sebaceous gland secretion regulation, more time of hyaluronic acid reinfusion in patients with wrinkles, but also to combat calvitie and hair loss, resulting in miofibroblasts.

Cellular regeneration is accomplished by exposure to the electromagnetic field obtained with the system according to the invention and consists of exposure to the electromagnetic field with parameters obtained therefrom, daily of one hour each for cosmetic applications and respectively of two hours each, for applications in the medical field for 50-100 hours for cosmetic applications, and 200-300 hours for medical applications.

## Claims

1. A modular electromagnetic system for cellular regeneration comprising:

   a patient positioning means (A) being a chair or bed;
   an upper coil-applicator (B) and a lower coil-applicator (C) adapted to apply an electromagnetic field to a desired area,
   the applicators (B, C) having the same construction and each comprising a magnetic core (13) around which some coils (14) are wounded which are mounted inside a rectangular or circular frame (15); the coils being adapted to generate a uniform electromagnetic field with induction comprised between 0, 7-0,8 mT;
   a support and displacement platform (D) of the upper coil-applicator (B) and of the lower coil-applicator (C); the platform (D) being adapted to adjust the position of the upper coil-applicator (B) and the lower coil-applicator (C) relative to the desired area; and a device (E) for generating the electromagnetic field;

   **characterized in that**:

   - said support and displacement platform (D) consists of a guiding tower (2) having at the top an upper platform (3) that supports and displaces the upper coil-applicator (B), and at the bottom a lower platform (4) that supports and displaces the lower coil-applicator (C); and wherein:

     - in order to allow the upper coil-applicator (B) to be raised or lowered by the upper support (3) and to be moved over the entire width of the tower (2), the upper support (3) is mounted at the top of the tower (2) by means of a guiding assembly (5) provided with a ball guide rail (6), movement of the rail (6) being achieved by means of an electric step by step motor, the lower support (4) being mounted at the bottom of the tower (2) by means of a guide assembly (10) provided with a guide rail (11) which is connected to the upper rail (6) for moving the upper support (3) so that with the displacement of the upper support (3) the lower applicator (C) moves; and

- said device (E) is adapted to generate low frequency current signals, providing a current in the range of 0 - 600 mA, settable in 50 mA steps, frequency ranging from 7 to 8 Hz, preferably 7.69 Hz; said device (E) being a low frequency sinusoidal/sine current generator in the range of 2 Hz to 25 Hz with seven outputs with a maximum output level of 600mA root mean square, RMS, at each output, maximum output voltage of 24 V RMS, built around a microprocessor (M) and a Digital Current Synthesizer DDS, adapted to directly generate precise sinusoidal signals with a variation from 2 to 50 Hz of a high precision and stability, driven by the processor (M) entering some amplifier units (AMPL1-7) *in class D* with an adjustable current which varies from 1 to 400 mA *and driver circuit dedicated with* pulse width modulator (PWM); where both the frequency and the current are continuously controlled by the microprocessor (M), the outputs of the amplifier units (AMPL1-7) being applied to the relevant terminals of the device (E) which is connected to the coils (14), *through a current control circuit (REGL/**BM**) for the output currents adjustment, which is realized with digital potentiometers by which the sinusoidal signal, representing the level of the output current to the modulator* (PWM) *is regulated.*

**2.** The modular electromagnetic system according to claim 1, **characterized by that** the upper support (3) can also be moved manually.

**3.** The modular electromagnetic system according to claim 1, **characterized by that** the upper coil-applicator (B) is fastened by means of an upper fixing frame (8) to the upper support (3).

**4.** The modular electromagnetic system according to claim 1, **characterized by that** the upper support (3) is fastened to the tower (2) by means of ball bearings (9) allowing the lower frame (8) to be lowered/lifted.

**5.** The modular electromagnetic system according to any one of claims 1 to 4, **characterized by that** the tower (2), the upper support (30) and the lower support (4) are made of glass-tectololite material so as not to deform the magnetic field around coil-applicators (B) and (C) respectively.

**6.** The modular electromagnetic system according to any of claims 1 to 6, **characterized by that** all the components of the system are modular, which allows them to be mounted and dismantled at the patient's home, at the workplace or at other places required by the patient.

**Patentansprüche**

**1.** Modulares elektromagnetisches System, umfassend eine Patientenpositionierungseinrichtung (A), die ein Stuhl oder ein Bett ist, einen oberen Spulenapplikator (B) und einen unteren Spulenapplikator (C), die dazu ausgelegt sind, ein elektromagnetisches Feld an einen gewünschten Bereich anzulegen, wobei die Applikatoren (B, C) die gleiche Konstruktion aufweisen und jeweils einen Magnetkern (13) umfassen, um den einige Spulen (14) gewickelt sind, die in einem rechteckigen oder kreisförmigen Rahmen (15) montiert sind; die Spulen werden ausgelegt, um ein gleichmäßiges elektromagnetisches Feld mit einer Induktion zwischen 0,7 und 0,8 mT zu erzeugen eine Stütz- und Verschiebungsplattform (D) des oberen Spulenapplikators (B) und des unteren Spulenapplikators (C); wobei die Plattform (D) angepasst ist, um die Position des oberen Spulenapplikators (B) und des unteren Spulenapplikators (C) relativ zum gewünschten Bereich einzustellen; und eine Vorrichtung (E) zum Erzeugen des elektromagnetischen Feldes; **dadurch gekennzeichnet, dass**:

- Die Stütz- und Verschiebungsplattform (D) besteht aus einem Führungsturm (2), der an dem oberen Teil eine obere Plattform (3) aufweist, die den oberen Spulenapplikator (B) stützt und verschiebt, und an dem unteren Teil eine untere Plattform (4) aufweist, das den unteren Spulenapplikator (C) stützt und verschiebt; und worin:

- der obere Träger (3), damit der obere Spulenapplikator (B) durch den oberen Träger (3) angehoben oder abgesenkt und über die gesamte Breite des Turms (2) bewegt werden kann, an dem oberen Teil des Turms (2) mittels einer Führungsanordnung (5) montiert ist, die mit einer Kugelführungsschiene (6) versehen ist, wobei die Verschiebung der Schiene (6), mittels eines elektrischen Schritt-für-Schritt-Motors erzielt wird, der untere Träger (4) am Boden des Turms (2) mittels einer Führungsanordnung (10) montiert ist, die mit einer Führungsschiene (11) versehen ist, die mit der oberen Schiene (6) zum Verschieben des oberen Trägers (3) verbunden ist, so dass der untere Applikator (C) sich mit der Verschiebung des oberen Trägers (3) bewegt; und

- die genannte Vorrichtung (E) zur Erzeugung niederfrequenter Stromsignale ausgelegt ist und einen Strom im Bereich von 0 bis 600 mA liefert, der in Schritten von 50 mA einstellbar ist, wobei die Frequenz im Bereich von 7 bis 8 Hz, vorzugsweise bei 7,69 Hz liegt;
- wobei die genannte Vorrichtung (E) ein Niederfrequenz-Sinusgenerator in dem Frequenzbereich von 2 Hz bis 25 Hz mit **sieben Ausgängen** mit einem maximalen Ausgangspegel von 600 mA Effektivwert, RMS, an jedem Ausgang, einer maximalen Ausgangsspannung von 24 V RMS ist, um einen Mikroprozessor (M) und einen digitalen Synthesizer DDS aufgebaut ist, der ausgelegt ist, um präzise sinusförmige Signale mit einer Variation von 2 bis 50 Hz mit hoher Präzision und Stabilität direkt zu erzeugen und von dem Prozessor (M) gesteuert ist, wobei diese in Verstärkereinheiten ( AMPL1-7) *in Klasse D* gelangen, mit einem einstellbaren Strom, der von 1 bis 400 mA variiert, und *einer Treiberschaltung, die mit einem Pulsweitenmodulator (PWM) ausgestattet ist,* wobei sowohl die Frequenz als auch der Strom kontinuierlich vom Mikroprozessor (M) gesteuert werden, wobei die Ausgänge der Verstärkereinheiten (AMPL1-7) an die relevanten Anschlüsse der Vorrichtung (E), die mit den Spulen (14) verbunden ist, *über eine Stromsteuerschaltung (REGL/**BM**) angelegt werden, um die Ausgangsströme einzustellen, was mit digitalen Potentiometern bewerkstelligt wird, durch welche das sinusförmige Signal, das den Pegel des Ausgangsstroms am Modulator (PWM) darstellt, geregelt wird.*

2. Modulares elektromagnetisches System nach Anspruch 1, **dadurch gekennzeichnet, dass** der obere Träger (3) auch manuell bewegt werden kann.

3. Modulares elektromagnetisches System nach Anspruch 1, **dadurch gekennzeichnet, dass** der obere Spulenapplikator (B) mittels eines oberen Befestigungsrahmens (8) am oberen Träger (3) befestigt ist.

4. Modulares elektromagnetisches System nach Anspruch 1, **dadurch gekennzeichnet, dass** der obere Träger (3) mittels Kugellagern (9) am Turm (2) befestigt ist, so dass der untere Rahmen (8) abgesenkt / angehoben werden kann.

5. Modulares elektromagnetisches System nach einem der Ansprüche 1 bis 4, **dadurch gekennzeichnet, dass** der Turm (2), der obere Träger (30) und der untere Träger (4) aus Glass-tectotolite-Material bestehen, um das Magnetfeld um die Spulenapplikatoren (B) bzw. (C) herum nicht zu verformen.

6. Modulares elektromagnetisches System nach einem der Ansprüche 1 bis 6, **dadurch gekennzeichnet, dass** alle Komponenten des Systems modular aufgebaut sind, wodurch sie zu Hause bei dem Patienten, am Arbeitsplatz oder an anderen von dem Patienten gewünschten Stellen montiert und demontiert werden können.

## Revendications

1. Système électromagnétique modulaire comprenant un moyen de positionnement du patient (A) étant une chaise ou un lit, un applicateur à bobine supérieur (B) et un applicateur à bobine inférieur (C) adaptés pour appliquer un champ électromagnétique à une zone souhaitée, les dit applicateurs (B, C) ayant la même construction et comprenant chacun un noyau magnétique (13) autour duquel sont enroulées des bobines (14) des applicateurs qui sont montées à l'intérieur d'un cadre rectangulaire ou circulaire (15); les bobines des applicateurs étant adaptées pour générer un champ électromagnétique uniforme avec une induction comprise entre 0,7 et 0,8 mT une plate-forme de support et de déplacement (D) du applicateur à bobine supérieur (B) et du applicateur à bobine inférieur (C); la plate-forme (D) étant adaptée pour régler la position de l'applicateur à bobine supérieur (B) et de l'applicateur à bobine inférieur (C) par rapport à la zone souhaitée; et un dispositif (E) pour générer le champ électromagnétique; **caractérisé en ce que**:

- ladite plate-forme de support et de déplacement (D) est constituée d'une tour de guidage (2) comportant en haut une plate-forme supérieure (3) qui supporte et déplace l'applicateur à bobine supérieur (B), et en bas une plate-forme inférieure (4) qui supporte et déplace le applicateur inférieur (C); et dans lequel:

- afin de permettre à l'applicateur à bobine supérieure (B) d'être relevée ou abaissée par le support supérieur (3) et d'être déplacée sur toute la largeur de la tour (2), le support supérieur (3) est monté au sommet de la tour (2) au moyen d'un ensemble de guidage (5) pourvu d'un rail de guidage à billes (6), le déplacement du rail (6) étant réalisé au moyen d'un moteur électrique pas à pas,

le support inférieur (4) étant monté au bas de la tour (2) au moyen d'un ensemble de guidage (10) pourvu d'un rail de guidage (11) qui est relié au rail supérieur (6) pour déplacer le support supérieur (3) de sorte qu'avec le déplacement du support supérieur (3) l'applicateur inférieur (C) se déplace; et

- ledit dispositif (E) pour générer le champ électromagnétique est adapté pour générer des signaux de courant basse fréquence, fournissant un courant dans la plage de 0 à 600 mA, réglable par pas de 50 mA, fréquence allant de 7 à 8 Hz, de préférence 7,69 Hz;

- ledit dispositif (E) pour générer le champ électromagnétique étant un générateur de courant sinusoïdal basse fréquence dans la gamme de 2 Hz à 25 Hz avec sept sorties avec un niveau de sortie maximal de 600mA moyenne quadratique, RMS, à chaque sortie, tension de sortie maximale de 24 V RMS, construit autour d'un microprocesseur (M) et d'un synthétiseur de courant numérique DDS (*Digital Current Synthesizer*), adapté pour générer directement des signaux sinusoïdaux précis avec une variation de 2 à 50 Hz d'une haute précision et stabilité, pilotés par le processeur (M) entrant dans certains amplificateurs (AMPL1-7) en classe D avec un courant réglable qui varie de 1 à 400 mA, et un circuit d'attaque dédié avec un modulateur de largeur d'impulsion (PWM), où la fréquence et le courant sont contrôlés en continu par le microprocesseur (M), les sorties de les unités d'amplification (AMPL1-7) étant appliquées aux bornes correspondantes du dispositif (E) qui est connecté aux bobines (14) des applicateurs, par un circuit de commande de courant (REGL / BM) pour le réglage des courants de sortie, qui est réalisé avec potentiomètres numériques par lesquels le signal sinusoïdal, représentant le niveau du courant de sortie vers le modulateur (PWM), est régulé.

2. Système électromagnétique modulaire selon la revendication 1, **caractérisé en ce que** le support supérieur (3) peut également être déplacé manuellement.

3. Système électromagnétique modulaire selon la revendication 1, **caractérisé en ce que** l'applicateur à bobine supérieur (B) est fixée au moyen d'un cadre de fixation supérieur (8) au support supérieur (3).

4. Système électromagnétique modulaire selon la revendication 1, **caractérisé en ce que** le support supérieur (3) est fixé à la tour (2) au moyen de roulements à billes (9) permettant d'abaisser / soulever le cadre inférieur (8).

5. Système électromagnétique modulaire selon l'une quelconque des revendications 1 à 4, **caractérisé en ce que** la tour (2), le support supérieur (3) et le support inférieur (4) sont en matériau verre-tectololite afin de ne pas se déformer le champ magnétique autour des dite applicateurs (B) et (C)/ à bobine supérieur et inférieur (B, C), respectivement.

6. Système électromagnétique modulaire selon l'une quelconque des revendications 1 à 6, **caractérisé en ce que** tous les composants du système sont modulaires, ce qui permet leur montage et démontage au domicile du patient, sur le lieu de travail ou à d'autres endroits requis par le patient.

a)

Fig. 1

b)

Fig. 1b

Fig. 1c

FIG. 1d

**Fig. 2**

Fig. 3

a)

b)

SECTION A-A
SCALE 1 / 4

Fig. 4

6

A

A

a)

b)

c)

SECTION A-A
SCALE 3 : 1

**Fig. 5**

c)

15

a)                                    b)

Fig. 6

Fig. 7

Figure 8

Figura 8

Figure 9

28

**Figure 10**

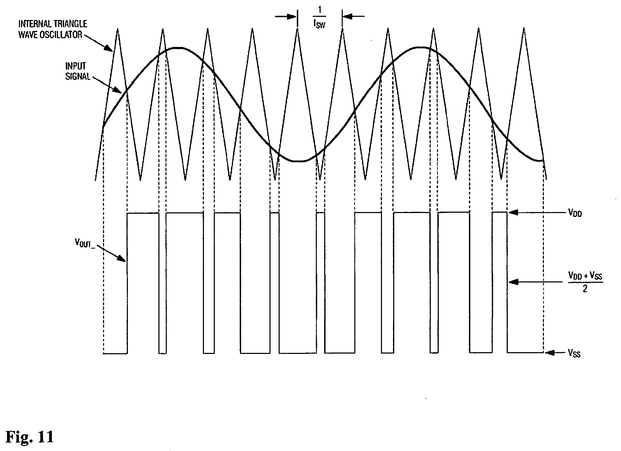

Fig. 11

**Fig. 12**

**EP 3 612 270 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- RO 131088 A1
- RO 130214 A1
- RO 130324 A1
- RO 131383 A0
- WO 200904215 A1
- RO 121463 B1
- WO 2017055471 A1, MAYR Winfried